(19) Europäisches Patentamt
European Patent Office
Office européen des brevets

(11) Veröffentlichungsnummer: **0 094 025**
**A1**

(12) # EUROPÄISCHE PATENTANMELDUNG

(21) Anmeldenummer: **83104388.0**

(22) Anmeldetag: **04.05.83**

(51) Int. Cl.³: **C 01 B 33/28**, B 01 J 29/28

(30) Priorität: **08.05.82 DE 3217322**

(43) Veröffentlichungstag der Anmeldung: **16.11.83**
**Patentblatt 83/46**

(84) Benannte Vertragsstaaten: **BE DE FR GB IT NL**

(71) Anmelder: **HOECHST AKTIENGESELLSCHAFT,**
**Postfach 80 03 20, D-6230 Frankfurt am Main 80 (DE)**

(72) Erfinder: **Baltes, Herbert, Dr., Johannesallee 24,**
**D-6230 Frankfurt am Main 80 (DE)**
Erfinder: **Litterer, Heinz, Dr., Albert-Schweitzer-Allee 39,**
**D-6200 Wiesbaden (DE)**
Erfinder: **Leupold, Ernst Ingo, Dr., Am Zäunefeld 15,**
**D-6392 Neu-Anspach (DE)**
Erfinder: **Wunder, Friedrich, Dr., Jahnstrasse 46,**
**D-6093 Flörsheim am Main (DE)**

(54) **Kristalline Aluminosilicat-Zeolithe, Verfahren zu ihrer Herstellung sowie ihre Verwendung.**

(57) Die Erfindung betrifft kristalline Aluminosilicat-Zeolithe, ein Verfahren zu deren Herstellung, sowie ihre Verwendung. Zur Herstellung wird eine Mischung aus Silicium-, Aluminium-, Natrium-, Kalium-, Ammoniumverbindungen und Wasser in spezifizierten Mengenverhältnissen hergestellt und in einem geschlossenen Gefäß erhitzt. Die Zeolithe finden Verwendung als Katalysatoren bei der Herstellung von $C_2$-$C_4$-Olefinen aus Methanol.

EP 0 094 025 A1

HOECHST AKTIENGESELLSCHAFT HOE 82/F 098 Dr.MA/cr

Kristalline Aluminosilicat-Zeolithe, Verfahren zu ihrer Herstellung sowie ihre Verwendung

Als Zeolithe bezeichnet man vor allem kristalline Aluminosilicate, bei denen durch eine dreidimensionale Verknüpfung von $SiO_4$- und $AlO_4$-Tetraedern regelmäßige Strukturen mit Hohlräumen und Poren entstehen. Im hydratisierten Zustand sind diese Poren und Hohlräume mit Wasser gefüllt. Dieses läßt sich ohne Beeinflussung der Kristallstruktur entfernen oder durch andere Moleküle ersetzen. Die negativen Ladungen der $AlO_4$-Tetraeder werden durch Kationen kompensiert. Diese können, wenn gewünscht, gegen andere Kationen ausgetauscht werden. Die geschilderten Eigenschaften ermöglichen die Verwendung der Zeolithe als Ionenaustauscher, Adsorbentien und Katalysatoren (D.W. Breck: Zeolite Molecular Sieves, 1974).

Zeolithe des X-, Y-, Mordenit-, Erionit- und Offretit-Typs beispielsweise besitzen als Katalysatoren für Umwandlungsreaktionen von Kohlenwasserstoffen wie Cracken, Hydrocracken oder Isomerisierungen beträchtliches technisches Interesse. Zeolithe vom Pentasil-Typ (z.B. Zeolith ZSM-5) gewinnen als Katalysatoren für die Umwandlung von Methanol zu Kohlenwasserstoffen steigende Bedeutung.

Aufgrund der zahlreichen Einsatzmöglichkeiten als Katalysatoren besteht großes Interesse an neuen Zeolithen mit spezifischen katalytischen Eigenschaften.

Gegenstand der Erfindung sind kristalline Aluminosilicat-Zeolithe, die dadurch gekennzeichnet sind, daß sie

a) Silicium, Aluminium, Natrium, Kalium und eine organische Ammoniumverbindung in folgendem Verhältnis enthalten

$SiO_2$: (0,02 - 0,30)$Al_2O_3$: (0,05 - 0,30)/$Na_2O$ + $K_2O$/:
(0,01 - 0,30)$R_2O$,

ausgedrückt in Molverhältnissen von Oxiden, wobei R Ammoniumreste der allgemeinen Formeln $(HOCH_2CH_2)_4N$, $(HOCH_2CH_2)_3R^1N$ oder $(HOCH_2CH_2)_2R^1R^2N$ bezeichnet und die Reste $R^1$ und $R^2$ gleich oder verschieden sein können und Alkyl, substituiertes Alkyl, Cycloalkyl, substituiertes Cycloalkyl, Aryl, substituiertes Aryl oder Wasserstoff bedeuten

und

b) im Röntgenbeugungsdiagramm die in Tabelle 1 aufgeführten charakteristischen Signale aufweisen:

Tabelle 1

| Netzebenenabstände d/ Å / | relative Intensität $I/I_o$ |
|---|---|
| 11,5 ± 0,3 | stark bis sehr stark |
| 9,2 ± 0,2 | schwach |
| 7,6 ± 0,2 | schwach bis mittel |
| 6,6 ± 0,1 | mittel bis stark |
| 6,3 ± 0,1 | schwach |
| 5,7 ± 0,1 | schwach |
| 5,35 ± 0,1 | schwach |
| 4,56 ± 0,1 | schwach bis mittel |
| 4,32 ± 0,1 | stark |
| 4,16 ± 0,1 | schwach |
| 3,81 ± 0,1 | mittel bis stark |
| 3,75 ± 0,1 | stark bis sehr stark |
| 3,59 ± 0,1 | stark bis sehr stark |
| 3,30 ± 0,1 | mittel |
| 3,15 ± 0,1 | mittel |
| 2,86 ± 0,1 | stark bis sehr stark |
| 2,80 ± 0,1 | schwach bis mittel |
| 2,67 ± 0,1 | schwach bis mittel |
| 2,49 ± 0,1 | schwach bis mittel |

Hierbei bedeutet $I_o$ die Intensität des stärksten Signals. Für die Angabe der Intensitäten in Tabelle 1 gilt:

| relative Intensität | $100\ I/I_o$ |
|---|---|
| sehr stark | 80 - 100 |
| stark | 50 - 80 |
| mittel | 20 - 50 |
| schwach | 0 - 20 |

Vorzugsweise besitzen die erfindungsgemäßen Zeolithe die folgende Zusammensetzung, ausgedrückt in Molverhältnissen der Oxide:

$SiO_2$: (0,08 - 0,18)$Al_2O_3$: (0,05 - 0,30) [$Na_2O$ + $K_2O$]: (0,01 - 0,30)$R_2O$.

Dabei hat R die oben angegebene Bedeutung, vorzugsweise ist R = $(HOCH_2CH_2)_3R^1N$.

$R^1$ und $R^2$ haben die oben angegebene Bedeutung, vorzugsweise sind sie Alkylreste mit maximal jeweils fünf C-Atomen oder Wasserstoff, insbesondere Methyl, Ethyl oder Wasserstoff.
$R^1$ und $R^2$ können verschieden sein,
vorzugsweise gilt jedoch $R^1$ = $R^2$,
insbesondere $R^1$ = $R^2$ = Methyl.

Die erfindungsgemäßen neuen Zeolithe besitzen eine dem Erionit (L.W. Staples, J.A. Gard, Mineralogical Magazine, Band 32, Jahrgang 1959, Seite 261 f) bzw. den synthetischen Zeolithen T (US-PS 2 950 952) und ZSM-34 (DE-OS 2 749 024) ähnliche Struktur, unterscheiden sich jedoch von diesen in der Zusammensetzung, den Sorptionseigenschaften und dem katalytischen Verhalten.

Die erfindungsgemäßen Zeolithe lassen sich herstellen, indem man eine Ammoniumverbindung RX mit Aluminium-, Silicium-, Natrium-, Kaliumverbindungen und Wasser mischt und in einem geschlossenen Gefäß erhitzt. Dabei hat R die oben angegebene Bedeutung. Dem Gemisch können darüberhinaus vor dem Erhitzen Impfkristalle zugesetzt werden.

Die Ausgangsverbindungen werden im allgemeinen in folgendem Verhältnis eingesetzt, ausgedrückt in Molverhältnissen der Oxide:

0094025

$SiO_2 : (0,02 - 0,30) Al_2O_3 : (0,02 - 0,4) Na_2O : (0,02 - 0,30) K_2O : (0,02 - 0,5) R_2O : (10 - 90) H_2O$,

vorzugsweise im Verhältnis

$SiO_2 : (0,04 - 0,18) Al_2O_3 : (0,10 - 0,30) Na_2O : (0,04 - 0,20) K_2O : (0,10 - 0,40) R_2O : (10 - 40) H_2O$.

Als Ammoniumverbindung RX können alle wasserlöslichen Salze von R eingesetzt werden. X kann beispielsweise bedeuten: Hydroxyl, Chlorid, Bromid, Jodid, Sulfat, Phosphat, Sulfonat, Carboxylat, Carbonat und Sulfit.

Die Ammoniumverbindung RX kann als Substanz eingesetzt werden. Vorzugsweise wird sie jedoch in situ im Reaktionsgemisch erzeugt, indem man ein Gemisch aus Triethanolamin und/oder Diethanolamin einerseits und einer Verbindung der allgemeinen Formel $R^1Y$ andererseits einsetzt, wobei $R^1$ die oben angegebene Bedeutung hat. Y ist im allgemeinen Hydroxyl, Monoalkylsulfat, Halogenid oder Sulfonat, insbesondere Hydroxyl.

$R^1Y$ ist vorzugsweise Methanol, Ethanol, Propanol, Butanol, Ethylenglykol, 1,2-Propylenglykol, Dimethylsulfat, Diethylsulfat, Methyljodid, Ethyljodid, Propyljodid, p-Toluolsulfonsäuremethylester, p-Toluolsulfonsäureethylester oder p-Toluolsulfonsäurepropylester. Insbesondere ist $R^1Y$ Methanol, Ethanol oder Ethylenglykol. Das Molverhältnis $R^1Y$ zu Amin (Triethanolamin und/oder Diethanolamin) beträgt im allgemeinen 0,5 bis 20, vorzugsweise 1 bis 10, insbesondere 4 bis 10.

Die erfindungsgemäßen Zeolithe lassen sich jedoch auch in Abwesenheit einer Verbindung der allgemeinen Formel $R^1Y$, d.h. nur in Gegenwart von Triethanolamin und/oder

Diethanolamin mit zufriedenstellender Kristallinität herstellen.

Als Silicium-, Aluminium-, Natrium- und Kaliumverbindungen können beispielsweise eingesetzt werden: Kieselsäuregel, Kaliumsilicat, Natriumsilicat, Natriumaluminat, Kaliumaluminat, Aluminiumhalogenide, Aluminiummetahydroxid, Kaliumhydroxid, Kaliumsulfat, Kaliumhalogenide, Natriumhydroxid, Natriumsulfat, Natriumhalogenide. Aber auch andere Silicium-, Aluminium-, Kalium- und Natriumverbindungen eignen sich für die Herstellung der erfindungsgemäßen Zeolithe.

Das Gemisch der jeweils gewählten Verbindungen mit Wasser wird im allgemeinen 18 bis 1000 Stunden, vorzugsweise 24 bis 500 Stunden lang auf eine Temperatur zwischen 80 und 200°C, vorzugsweise zwischen 110 und 160°C, in einem geschlossenen Gefäß erhitzt.

Die gebildeten Zeolithe werden in üblicher Weise, z.B. durch Filtration, isoliert, gewaschen und getrocknet. Sie können nach bekannten Methoden in die katalytisch aktiven Formen überführt werden, z.B. durch Kalzinierung und/oder Ionenaustausch (D.W. Breck, Zeolite Molecular Sieves, 1974).

Die erfindungsgemäßen Zeolithe zeichnen sich nach ihrer Überführung in die katalytisch aktiven Formen insbesondere aus durch eine hohe Selektivität und durch eine geringe Koksabscheidung bei der Umwandlung von Methanol in niedere Olefine. Es ist überraschend, daß man mit Hilfe der angegebenen Methode überhaupt Zeolithe mit den erfindungsgemäßen Merkmalen erhält.

Die Erfindung soll durch die folgenden Beispiele erläutert werden, wobei die Beispiele aber in keiner Weise einschränkend sein sollen. Alle angegebenen Röntgenbeugungsdaten wurden mit einem computergesteuerten Pulverdiffraktometer D-500 der Firma Siemens aufgenommen. Es wurde Kupfer-K-$\alpha$-Strahlung verwandt.

Beispiel 1

11,2 g Natriumaluminat (54 Gew.-% $Al_2O_3$, 41 Gew.-% $Na_2O$), 5,9 g Natriumhydroxid, 5,3 g Kaliumhydroxid, 48,7 g Triethanolamin und 31 g Methanol werden in 150 ml Wasser gelöst. In diese Lösung werden 117 g 40 gew.-%iges kolloidales Kieselgel eingebracht. Die entstandene Mischung wird homogenisiert und 226 h im geschlossenen Gefäß auf 140°C erhitzt. Das entstandene Produkt wird abfiltriert, mit Wasser gewaschen und bei 120°C getrocknet.

Das Produkt besitzt das in der Tabelle 2 wiedergegebene Röntgenbeugungsmuster.

Die chemische Analyse ergibt folgende Zusammensetzung, ausgedrückt in Molverhältnissen von Oxiden:

$SiO_2$: 0,139 $Al_2O_3$ : 0,069 $Na_2O$ : 0,086 $K_2O$ : 0,057 $R_2O$, wobei R = $(HOCH_2CH_2)_3NCH_3$ ist.

Tabelle 2

| Netzebenenabstände d/Å/ | relative Intensität 100 $I/I_o$ |
|---|---|
| 11,4 | 93 |
| 9,1 | 4 |
| 7,54 | 15 |
| 6,58 | 68 |
| 6,27 | 6 |
| 5,70 | 4 |
| 5,33 | 3 |
| 4,98 | 4 |
| 4,55 | 12 |
| 4,31 | 56 |
| 4,16 | 5 |
| 3,81 | 26 |
| 3,75 | 100 |
| 3,57 | 67 |
| 3,30 | 23 |
| 3,14 | 45 |
| 2,92 | 3 |
| 2,86 | 61 |
| 2,84 | 78 |
| 2,81 | 18 |
| 2,67 | 24 |
| 2,50 | 5 |

Beispiel 2

11,2 g Natriumaluminat (54 Gew.-% $Al_2O_3$, 41 Gew.-% $Na_2O$), 5,9 g Natriumhydroxid, 5,3 g Kaliumhydroxid und 97,4 g Triethanolamin werden in 150 ml Wasser gelöst. In diese Lösung werden 117 g 40 gew.-%iges kolloidales Kieselgel und 0,5 g Impfkristalle aus Beispiel 1 eingebracht. Die entstandene Mischung wird homogenisiert und 192 h im geschlossenen Gefäß auf 150°C erhitzt. Das entstandene

Produkt wird abfiltriert, mit Wasser gewaschen und bei 120°C 24 h getrocknet.

Das Produkt zeigt die in Tabelle 1 angegebenen Röntgensignale. Ein Teil des Produktes wurde 24 h bei 540°C kalziniert. Das Röntgenbeugungsmuster des kalzinierten Produktes ist in der Tabelle 3 wiedergegeben. Es besitzt folgende chemische Zusammensetzung, ausgedrückt in Molverhältnissen von Oxiden:

$SiO_2$ : 0,081 $Al_2O_3$ : 0,089 $Na_2O$ : 0,039 $K_2O$.

Tabelle 3

| Netzebenenabstände d/Å/ | relative Intensität 100 $I/I_o$ |
|---|---|
| 11,3 | 74 |
| 9,1 | 5 |
| 7,53 | 15 |
| 6,56 | 56 |
| 6,25 | 4 |
| 5,69 | 4 |
| 5,32 | 3 |
| 4,54 | 10 |
| 4,30 | 56 |
| 4,16 | 7 |
| 3,80 | 25 |
| 3,73 | 100 |
| 3,55 | 66 |
| 3,28 | 22 |
| 3,14 | 33 |
| 2,86 | 51 |
| 2,83 | 75 |
| 2,65 | 16 |
| 2,46 | 16 |

Beispiel 3

2,24 g Natriumaluminat, 1,2 g Natriumhydroxid, 1,1 g Kaliumhydroxid, 9,7 g Triethanolamin und 12 g Ethylenglykol werden in 30 ml Wasser gelöst. In diese Lösung werden 23,4 g 40 gew.-%iges kolloidales Kieselgel eingebracht. Die entstandene Mischung wird homogenisiert und 336 h auf 110°C erhitzt. Nach einer Aufarbeitung wie in Beispiel 1 erhält man ein kristallines Produkt, dessen Röntgenbeugungsdaten den in Tabelle 1 angegebenen entsprechen.

Die chemische Analyse ergibt folgende Zusammensetzung, ausgedrückt in Molverhältnissen von Oxiden:

$SiO_2$ : 0,106 $Al_2O_3$ : 0,041 $Na_2O$ : 0,044 $K_2O$ : 0,048 $R_2O$, wobei R = $(HOCH_2CH_2)_4N$ ist.

Beispiel 4

11,2 g Natriumaluminat, 5,9 g Natriumhydroxid, 5,3 g Kaliumhydroxid, 34,3 g Diethanolamin und 62 g Methanol werden in 150 ml Wasser gelöst. In diese Lösung werden 117 g 40 gew.-%iges kolloidales Kieselgel eingebracht. Die entstandene Mischung wird homogenisiert und 192 h im geschlossenen Gefäß auf 150°C erhitzt. Das entstandene Produkt wird abfiltriert, mit Wasser gewaschen und bei 140°C getrocknet. Das Produkt besitzt das in Tabelle 1 angegebene Röntgenbeugungsmuster.

Beispiel 5

2,0 g Natriumaluminat, 1,0 g Natriumhydroxid, 1,0 g Kaliumhydroxid, 14,6 g Triethanolamin und 9,0 g Ethanol werden in 30 ml Wasser gelöst. In diese Lösung werden 24,0 g 40 gew.-%iges kolloidales Kieselgel eingebracht.

0094025

Die entstandene Mischung wird homogenisiert und 192 h auf 150°C erhitzt. Nach einer Aufarbeitung wie in Beispiel 1 erhält man ein kristallines Produkt, dessen Röntgendaten den in Tabelle 1 angegebenen entsprechen.

Beispiel 6

2,24 g Natriumaluminat, 1,2 g Natriumhydroxid, 1,1 g Kaliumhydroxid, 9,7 g Triethanolamin und 8,3 g Dimethylsulfat werden in 30 ml Wasser gelöst. In diese Lösung werden 23,4 g 40 gew.-%iges kolloidales Kieselgel und 0,5 g Impfkristalle aus Versuch 1 eingebracht. Die entstandene Mischung wird homogenisiert und 192 h auf 160°C erhitzt. Nach einer Aufarbeitung wie in Beispiel 1 erhält man ein kristallines Produkt, dessen Röntgendaten den in Tabelle 1 angegebenen entsprechen.

0094025
HOE 82/F 098

PATENTANSPRÜCHE

1. Kristalline Aluminosilicat-Zeolithe, dadurch gekennzeichnet, daß sie

a) Silicium, Aluminium, Natrium, Kalium und eine organische Ammoniumverbindung in folgendem Verhältnis enthalten

$SiO_2$ : (0,02 - 0,30)$Al_2O_3$ : (0,05 - 0,30) [$Na_2O+K_2O$] : (0,01 - 0,30)$R_2O$,

ausgedrückt in Molverhältnissen von Oxiden, wobei R Ammoniumreste der allgemeinen Formeln $(HOCH_2CH_2)_4N$, $(HOCH_2CH_2)_3R^1N$ oder $(HOCH_2CH_2)_2R^1R^2N$ bezeichnet und die Reste $R^1$ und $R^2$ gleich oder verschieden sein können und Alkyl, substituiertes Alkyl, Cycloalkyl, substituiertes Cycloalkyl, Aryl, substituiertes Aryl oder Wasserstoff bedeuten

und

b) im Röntgenbeugungsdiagramm die folgenden charakteristischen Signale aufweisen:

| Netzebenenabstände d/Å | relative Intensität $I/I_o$ |
|---|---|
| 11,5 ± 0,3 | stark bis sehr stark |
| 9,2 ± 0,2 | schwach |
| 7,6 ± 0,2 | schwach bis mittel |
| 6,6 ± 0,1 | mittel bis stark |
| 6,3 ± 0,1 | schwach |
| 5,7 ± 0,1 | schwach |
| 5,35 ± 0,1 | schwach |
| 4,56 ± 0,1 | schwach bis mittel |
| 4,32 ± 0,1 | stark |
| 4,16 ± 0,1 | schwach |
| 3,81 ± 0,1 | mittel bis stark |
| 3,75 ± 0,1 | stark bis sehr stark |

Fortsetzung

| Netzebenenabstände d/$\overline{\,}$Å_7 | relative Intensität $I/I_o$ |
|---|---|
| 3,59 ± 0,1 | stark bis sehr stark |
| 3,30 ± 0,1 | mittel |
| 3,15 ± 0,1 | mittel |
| 2,86 ± 0,1 | stark bis sehr stark |
| 2,80 ± 0,1 | schwach bis mittel |
| 2,67 ± 0,1 | schwach bis mittel |
| 2,49 ± 0,1 | schwach bis mittel |

wobei $I_o$ die Intensität des stärksten Signals bedeutet.

2. Kristalline Aluminosilicat-Zeolithe nach Anspruch 1, dadurch gekennzeichnet, daß sie die folgende Zusammensetzung besitzen:

$SiO_2$ : (0,08 - 0,18)$Al_2O_3$ : (0,05 - 0,30)/$\overline{\,}$$Na_2O$ + $K_2O$_7 : (0,01 - 0,30) $R_2O$.

3. Kristalline Aluminosilicat-Zeolithe nach einem der Ansprüche 1 oder 2, dadurch gekennzeichnet, daß $R^1$ und $R^2$ Alkylreste mit maximal jeweils fünf C-Atomen oder Wasserstoff sind.

4. Kristalline Aluminosilicat-Zeolithe nach einem der Ansprüche 1 oder 2, dadurch gekennzeichnet, daß $R^1$ und $R^2$ Methyl, Ethyl oder Wasserstoff sind.

5. Kristalline Aluminosilicat-Zeolithe nach einem der Ansprüche 1 bis 4, dadurch gekennzeichnet, daß $R^1$ gleich $R^2$ ist.

6. Kristalline Aluminosilicat-Zeolithe nach einem der Ansprüche 1 oder 2, dadurch gekennzeichnet, daß $R^1$ und $R^2$ gleich Methyl sind.

7. Verfahren zur Herstellung von kristallinen Aluminosilicat-Zeolithen nach einem der Ansprüche 1 bis 6, dadurch gekennzeichnet, daß man eine Mischung aus Silicium-, Aluminium-, Natrium-, Kalium-, Ammoniumverbindungen und Wasser herstellt, die folgende Zusammensetzung hat, ausgedrückt in Molverhältnissen der Oxide:

$SiO_2$ : (0,02 - 0,30)$Al_2O_3$ : (0,02 - 0,40)$Na_2O$ :
(0,02 - 0,30)$K_2O$ : (0,02 - 0,50)$R_2O$:(10 - 90)$H_2O$

und diese Mischung in einem geschlossenen Gefäß erhitzt.

8. Verfahren nach Anspruch 7, dadurch gekennzeichnet, daß die zu erhitzende Mischung folgende Zusammensetzung hat, ausgedrückt in Molverhältnissen der Oxide:

$SiO_2$ : (0,04 - 0,18)$Al_2O_3$ : (0,10 - 0,30)$Na_2O$ :
(0,04 - 0,20)$K_2O$ : (0,10 - 0,40)$R_2O$ : (10-40)$H_2O$

9. Verfahren nach einem der Ansprüche 7 oder 8, dadurch gekennzeichnet, daß man anstelle einer Ammoniumverbindung eine äquivalente Menge an Triethanolamin und/oder Diethanolamin zusammen mit einer Verbindung der allgemeinen Formel $R^1Y$ einsetzt, wobei $R^1$ gleich Alkyl, substituiertes Alkyl, Cycloalkyl, substituiertes Cycloalkyl, Aryl, substituiertes Aryl oder Wasserstoff ist und Y gleich Hydroxyl, Monoalkylsulfat, Halogenid oder Sulfonat ist.

10. Verfahren nach Anspruch 9, dadurch gekennzeichnet, daß $R^1Y$ gleich Methanol, Ethanol, Propanol, Butanol Ethylenglykol, 1,2-Propylenglykol, Dimethylsulfat,

Diethylsulfat, Methyljodid, Ethyljodid, Propyljodid, p-Toluolsulfonsäuremethylester, p-Toluolsulfonsäureethylester, oder p-Toluolsulfonsäurepropylester ist.

11. Verfahren nach Anspruch 9, dadurch gekennzeichnet, daß $R^1Y$ gleich Methanol, Ethanol oder Ethylenglykol ist.

12. Verfahren nach einem der Ansprüche 7 oder 8, dadurch gekennzeichnet, daß man anstelle einer Ammoniumverbindung eine äquivalente Menge an Triethanolamin einsetzt.

13. Verfahren nach einem der Ansprüche 7 oder 8, dadurch gekennzeichnet, daß man anstelle einer Ammoniumverbindung eine äquivalente Menge an Diethanolamin einsetzt.

14. Verwendung von kristallinen Aluminosilicat-Zeolithen nach einem der Ansprüche 1 bis 6 als Katalysatoren bei der Herstellung von $C_2$ bis $C_4$-Olefinen aus Methanol.

0094025

Europäisches Patentamt

## EUROPÄISCHER RECHERCHENBERICHT

Nummer der Anmeldung

EP 83 10 4388

### EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| | | | C 01 B 33/28<br>B 01 J 29/28 |
| P,A | EP-A-0 057 049 (BP)<br>* Anspruch 9 * | | |
| P,A | EP-A-0 055 529 (ICI) | | |
| A | EP-A-0 002 900 (BP) | | |
| A | DE-A-2 924 870 (SNAMPROGETTI)<br>* Beispiel 6; Ansprüche 16, 17 * | | |
| D,A | DE-A-2 749 024 (MOBIL OIL) | | |
| D,A | US-A-2 950 952 (D.W. BRECK et al.) | | RECHERCHIERTE SACHGEBIETE (Int. Cl. 3)<br>C 01 B 33/00<br>B 01 J 29/00 |

Der vorliegende Recherchenbericht wurde für alle Patentansprüche erstellt.

| Recherchenort | Abschlußdatum der Recherche | Prüfer |
|---|---|---|
| BERLIN | 15-08-1983 | KESTEN W |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503. 03.82